# EUROPÄISCHE PATENTSCHRIFT

(11) **EP 3 023 896 B1**
(45) Veröffentlichungstag und Bekanntmachung des Hinweises auf die Patenterteilung: **20.02.2019**
(21) Anmeldenummer: 14194515.4
(22) Anmeldetag: 24.11.2014
(51) Int. Cl.: G06F 19/00

(54) **Verfahren zum Übertragen von medizinischen Datensätzen**
Method for transmitting medical data sets
Procédé de transmission de jeux de données médicales

(43) Veröffentlichungstag der Anmeldung: 25.05.2016
(73) Patentinhaber: Siemens Healthcare GmbH, 91052 Erlangen (DE)
(72) Erfinder: Schottlander, David, Kidlington, Oxfordshire OX5 1EN (GB); Goßler, Thomas, 91056 Erlangen (DE); Ukis, Vladyslav, 90489 Nürnberg (DE)

(56) Entgegenhaltungen:
- WO-A1-2013/188850
- US-A1- 2010 042 583

## Beschreibung

Die vorliegende Erfindung betrifft Verfahren zum Übertragen von medizinischen Datensätzen von einem internen Datenspeicher an einen externen Datenspeicher.

Medizinische Einrichtungen, wie beispielsweise Krankenhäuser, verwenden zunehmend Cloud-basierte Software-Lösungen. Diese Software-Lösungen erfordern ein Hochladen sensitive medizinischer Patientendaten in ein Datenzentrum. Das Hochladen wird mittels einer speziellen Hochlade-Software durchgeführt. Die Hochlade-Software lädt die Patientendaten ohne Anonymisierung an das Datenzentrum hoch. Daher muss das Krankenhaus eine Anonymisierung vor dem eigentlichen Hochladen durchführen, um die jeweiligen Datenschutzbestimmungen einzuhalten. Dieses Vorgehen ist fehleranfällig, zeitintensiv und schwer zu steuern. Insbesondere gelten in unterschiedlichen Ländern länderspezifische Datenschutzbestimmungen. Zudem kann nicht sichergestellt werden, dass die hochgeladenen Patientendaten in der Cloud verarbeitet werden können.

Die Druckschrift WO 2013/188850 A1 betrifft ein Netzwerkgerät und ein Peripheriegerät für eine Verschlüsselung und die Umwandlung eines medizinischen Bildes in ein sicheres und standardisiertes Bilddateiformat sowie eine Übertragung der verschlüsselten oder umgewandelte Bild an einen sicheren Server über ein Remote-Netzwerk.

Die Druckschrift US 2010/042583 A1 betrifft Systeme und Verfahren für die De-Identifikation von Daten.

Es ist die Aufgabe der vorliegenden Erfindung, Patientendatensätze mit geringen technischen Ressourcen automatisch derart anzupassen, dass unterschiedliche Datenschutzrichtlinien eingehalten werden.

Diese Aufgabe wird durch einen Gegenstand nach dem unabhängigen Anspruch gelöst. Vorteilhafte Ausführungsformen sind Gegenstand der abhängigen Ansprüche, der Beschreibung und der Zeichnungen.

Gemäß einem ersten Aspekt wird die Aufgabe durch ein Verfahren zum Übertragen von medizinischen Datensätzen gelöst, mit den Schritten eines Empfangens eines Patientendatensatzes aus einem internen Datenspeicher; eines Auswählens einer Anonymisierungseinstellung aus einem Satz von vorgegebenen Anonymisierungseinstellungen; eines Erzeugens eines anonymisierten Patientendatensatzes auf Basis der ausgewählten Anonymisierungseinstellung; und eines Übertragens des anonymisierten Patientendatensatzes an einen externen Datenspeicher. Dadurch wird beispielsweise der technische Vorteil erreicht, dass ein Satz von Anonymisierungseinstellungen verwendet wird, die an unterschiedliche Funktionen des externen Datenspeichers angepasst sein können. Zu diesem Zweck kann ein Satz von Eigenschaften häufig verwendeter Dienste des externen Datenspeichers verwendet werden, die für jede Anonymisierungseinstellung definiert sind. Zudem ist die Möglichkeit gegeben, eine Anonymisierungseinstellung während einem Software-Einsatz in einer medizinischen Einrichtung in Abhängigkeit des Einsatzortes des externen Datenspeichers auszuwählen.

In einer vorteilhaften Ausführungsform des Verfahrens wird der anonymisierte Patientendatensatz in dem externen Datenspeicher gespeichert. Dadurch wird beispielsweise der technische Vorteil erreicht, dass der Patientendatensatz dauerhaft verfügbar ist.

In einer weiteren vorteilhaften Ausführungsform des Verfahrens wird die Anonymisierungseinstellung automatisch auf Basis eines Standortes des externen Datenspeichers ausgewählt. Dadurch wird beispielsweise der technische Vorteil erreicht, dass am Standort des externen Datenspeichers zulässige Patientendatensätze vorliegen.

In einer weiteren vorteilhaften Ausführungsform des Verfahrens umfasst der anonymisierte Patientendatensatz eine logische Verknüpfung zu der ausgewählten Anonymisierungseinstellung. Dadurch wird beispielsweise der technische Vorteil erreicht, dass die Anonymisierungseinstellung anhand des anonymisierten Patientendatensatzes bestimmt werden kann.

In einer weiteren vorteilhaften Ausführungsform des Verfahrens ist der empfangene und/oder der anonymisierte Patientendatensatz eine DICOM-Datei. Dadurch wird beispielsweise der technische Vorteil erreicht, dass ein besonders geeignetes Format zum Speichern und verarbeiten der Patientendatensätze verwendet wird.

In einer weiteren vorteilhaften Ausführungsform des Verfahrens wird die Anonymisierungseinstellung in den anonymisierten Patientendatensatz eingebettet. Dadurch wird beispielsweise der technische Vorteil erreicht, dass die Anonymisierungseinstellung in dem anonymisierten Patientendatensatz enthalten ist.

In einer weiteren vorteilhaften Ausführungsform des Verfahrens wird der anonymisierte Patientendatensatz in dem internen Datenspeicher gespeichert. Dadurch wird beispielsweise der technische Vorteil erreicht, dass der anonymisierte Patientendatensatz zusätzlich gesichert wird.

In einer weiteren vorteilhaften Ausführungsform des Verfahrens werden die Daten des Patientendatensatzes, die beim Erzeugen des anonymisierten Patientendatensatzes anonymisiert werden, in einem zusätzlichen nicht-anonymisierten Datensatz gespeichert. Dadurch wird beispielsweise der technische Vorteil erreicht, dass die Daten trotz einer Anonymisierung weiterhin verfügbar sind.

In einer weiteren vorteilhaften Ausführungsform des Verfahrens wird eine Abbildung zwischen dem nicht-anonymisierten Datensatz und dem anonymisierten Patientendatensatz erzeugt. Dadurch wird beispielsweise der technische Vorteil erreicht, dass die Daten einem anonymisierten Patientendatensatz zugeordnet werden können.

In einer weiteren vorteilhaften Ausführungsform des Verfahrens wird die Abbildung verschlüsselt gespeichert. Dadurch wird beispielsweise der technische Vorteil erreicht, dass ein nicht autorisierter Zugriff auf die Abbildungsdaten verhindert wird.

In einer weiteren vorteilhaften Ausführungsform des Verfahrens übermittelt der externe Datenspeicher einen Dienstparameter, der anzeigt, welche Dienste von dem externen Datenspeicher bereitgestellt werden. Dadurch wird beispielsweise der technische Vorteil erreicht, dass das Verfahren diese Parameter für weitere Entscheidungsprozesse verwenden kann.

In einer weiteren vorteilhaften Ausführungsform des Verfahrens wird die Anonymisierungseinstellung auf Basis des Dienstparameters ausgewählt. Dadurch wird beispielsweise der technische Vorteil erreicht, dass je nach laufendem Dienst Patientendatensätze erzeugt werden, die von den Diensten verarbeitet werden können.

In einer weiteren vorteilhaften Ausführungsform des Verfahrens ist die Anonymisierungseinstellung deaktivierbar. Dadurch wird beispielsweise der technische Vorteil erreicht, dass die Patientendatensätze bei Bedarf unverändert übertragen werden können.

In einer weiteren vorteilhaften Ausführungsform des Verfahrens wird der anonymisierte Patientendatensatz vor einem Übertragen verschlüsselt. Dadurch wird beispielsweise der technische Vorteil erreicht, dass die Datensicherheit nochmals erhöht wird.

Gemäß einem zweiten Aspekt wird die Aufgabe durch ein Computersystem zum Übertragen von medizinischen Datensätzen gelöst, mit einer Empfangseinrichtung zum Empfangen eines Patientendatensatzes aus einem internen Datenspeicher; einer Auswahleinrichtung zum Auswählen einer Anonymisierungseinstellung aus einem vorgegebenen Satz von Anonymisierungseinstellungen; einer Erzeugungseinrichtung zum Erzeugen eines anonymisierten Patientendatensatzes auf Basis der ausgewählten Anonymisierungsregel; und einer Sendeeinrichtung zum Übertragen des anonymisierten Patientendatensatzes und des verschlüsselten Patientenidentifikationsdatensatzes an einen externen Datenspeicher. Dadurch werden die gleichen technischen Vorteile wie durch das Verfahren nach dem ersten Aspekt erreicht.

Ausführungsbeispiele der Erfindung sind in den Zeichnungen dargestellt und werden im Folgenden näher beschrieben.

Es zeigen:
- Fig. 1: ein Blockdiagramm eines Verfahrens.
- Fig. 2: eine Tabelle mit DICOM-Daten;
- Fig. 3: Eigenschaften eines Dosismanagement-Dienstes;
- Fig. 4: Eigenschaften eines Scanner-Nutzungsdienstes;
- Fig. 5: eine schematische Ansicht unterschiedlicher Patientendatensätze;
- Fig. 6: eine Darstellung der Logik;
- Fig. 7: ein Beispiel einer Benutzerschnittstelle;
- Fig. 8: ein weiteres Beispiel einer Benutzerschnittstelle;
- Fig. 9: ein weiteres Beispiel einer Benutzerschnittstelle; und
- Fig. 10: ein Computersystem zum Übertragen von medizinischen Datensätzen.

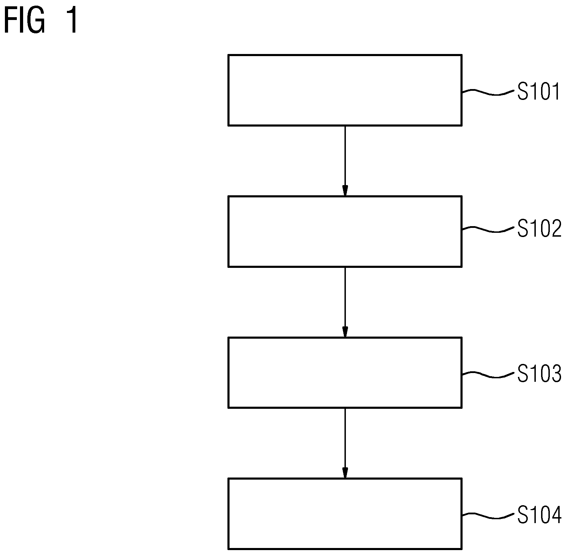

Fig. 1 zeigt ein Blockdiagramm des Verfahrens zum Übermitteln von medizinischen Datensätzen. In einem Schritt S101 wird zunächst ein Patientendatensatz aus einem internen Datenspeicher empfangen. Der interne Datenspeicher kann durch ein Bildablage- und Kommunikationssystem (PACS - Picture Archiving and Communication System) innerhalb eines Krankenhauses gebildet sein.

Im Anschluss wird in Schritt S102 eine Anonymisierungseinstellung aus einem Satz von vorgegebenen Anonymisierungseinstellungen ausgewählt. Das Auswählen kann automatisch in Abhängigkeit eines Standortes des externen Datenspeichers oder der Bearbeitungsfunktionen des externen Datenspeichers durchgeführt werden. In Schritt S103 wird ein anonymisierter Patientendatensatz auf Basis der ausgewählten Anonymisierungseinstellung erzeugt. Dabei werden bestimmte Daten des Patientendatensatzes anonymisiert, beispielsweise indem Patientenidentifikationsdaten entfernt werden oder durch Zufallsdaten ersetzt werden. Anschließend wird in Schritt S104 der anonymisierte Patientendatensatz an einen externen Datenspeicher Übermittelt. Der externe Datenspeicher wird beispielsweise durch eine Cloud im Internet gebildet.

Das Verfahren dient zum automatisierten Datenschutzmanagement auf einem Server, der medizinische Patientendatensätze von einem Krankenhaus in ein Cloud-Datencenter als externen Datenspeicher hoch lädt. Das Verfahren kann durch eine Hochlade-Software auf einem Server ausgeführt werden, die weltweit in einem beliebigen Krankenhaus verwendet wird und die automatisch lokal geltende Datenschutzbestimmungen in Abhängigkeit des Einsatzlandes und der verwendeten Cloud-Dienste unterstützt und anwendet.

Daneben kann technisch sichergestellt werden, dass die Anonymisierung oder Pseudonymisierung der Patientendaten durch die Hochlade-Software derart durchgeführt wird, dass die Cloud-Dienste die hochgeladenen Patientendaten verarbeiten können. Dadurch kann eine Situation verhindert werden, bei der die anonymisierten oder pseudonymisierten Patientendaten nicht durch die Cloud-Dienste verwendbar sind.

Der Satz an vorgegebenen Anonymisierungseinstellungen kann beispielsweise drei Anonymisierungseinstellungen umfassen, die in einem Großteil von Datenschutzfällen anwendbar sind. Dabei kann zwischen einer Standard-Anonymisierungseinstellung, einer strengen Anonymisierungseinstellung und einer sehr strengen Anonymisierungseinstellung unterschieden werden.

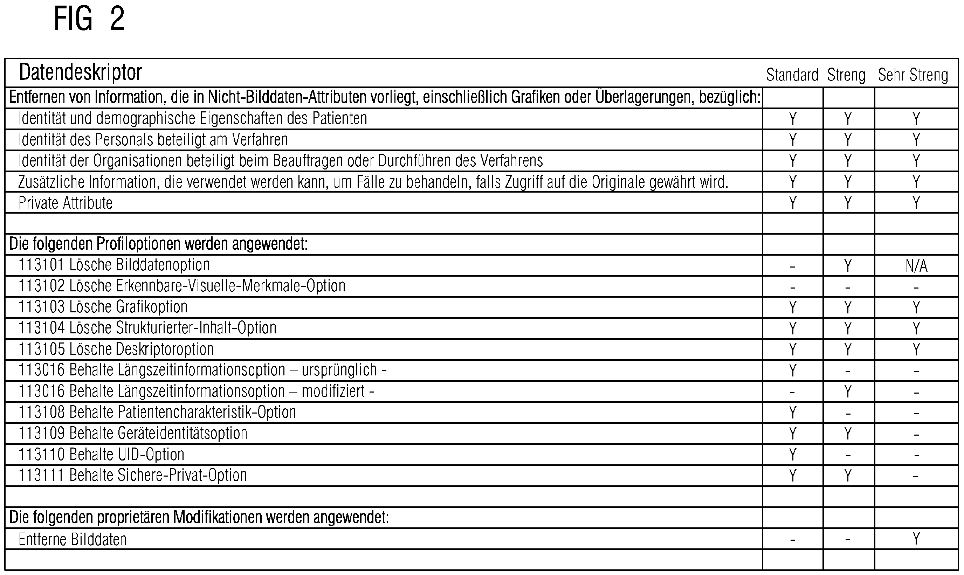

Fig. 2 zeigt eine Tabelle von DICOM-Daten, die von der medizinischen Einrichtung in die Cloud in Abhängigkeit der Anonymisierungseinstellung hochgeladen werden. Dabei wird zwischen drei vorgegebenen Anonymisierungseinstellungen unterschieden, d.h. einer Standard-Anonymisierungseinstellung, einer strengen Anonymisierungseinstellung und einer sehr strengen Anonymisierungseinstellung. Je nach ausgewählter Anonymisierungseinstellung wird der Patientendatensatz auf unterschiedliche Weise bearbeitet. Beispielsweise werden unter der sehr strengen Anonymisierungseinstellung Bilddaten aus den Patientendatensätzen entfernt, während dies bei der Standard-Anonymisierungseinstellung und der strengen Anonymisierungseinstellung nicht der Fall ist.

Die Cloud kann unterschiedliche Dienste zum Bearbeiten der Patientendatensätze bereitstellen. Beispielsweise ist es möglich, einen Dosismanagement-Dienst, einen Scanner-Nutzungsdienst oder einen Bildverteilungsdienst bereitzustellen.

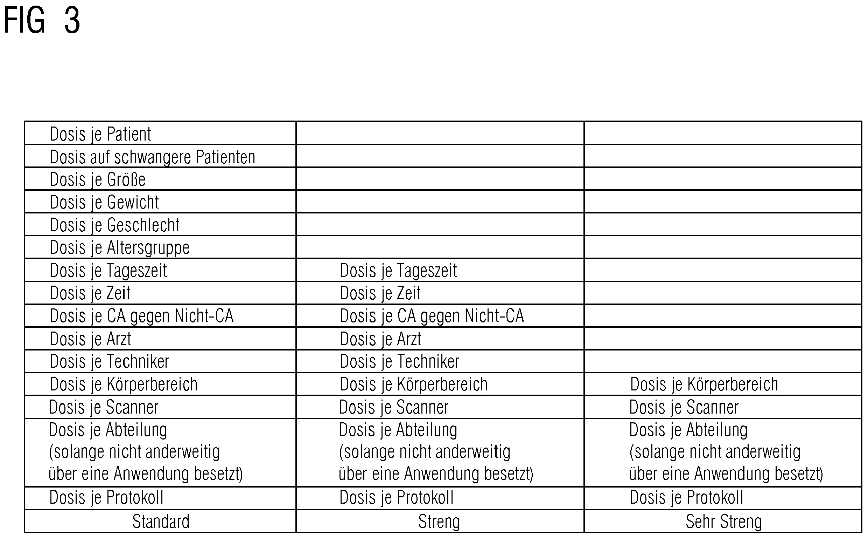

Fig. 3 zeigt die Eigenschaften eines Dosismanagement-Dienstes in Abhängigkeit der Anonymisierungseinstellung. Bei einer Standard- Anonymisierungseinstellung wird beispielsweise die Dosis je Patient ausgewertet, während dies bei einer strengen oder sehr strengen Anonymisierungseinstellung nicht durchgeführt wird.

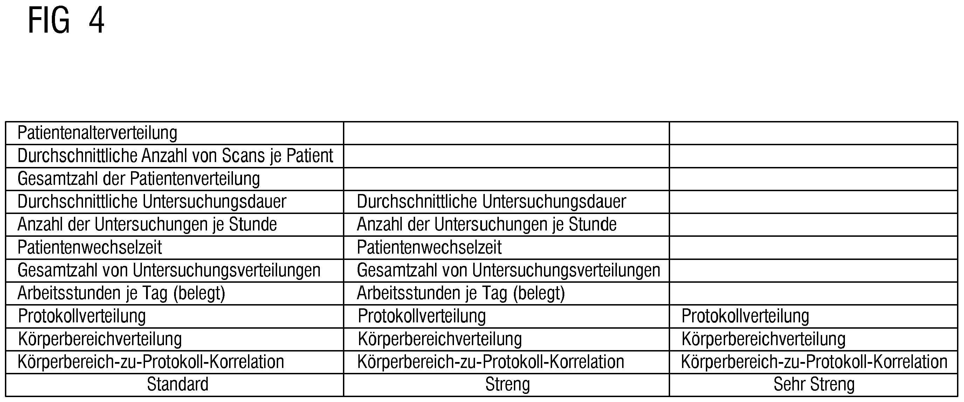

Fig. 4 zeigt die Eigenschaften eines Scanner-Nutzungsdienstes in Abhängigkeit der Anonymisierungseinstellung. Bei einer Standard- Anonymisierungseinstellung wird beispielsweise die Patientenaltersverteilung ausgewertet, während dies bei einer strengen oder sehr strengen Anonymisierungseinstellung nicht durchgeführt wird.

Der Bildverteilungsdienst ist vollständig lediglich in der Standard- Anonymisierungseinstellung verfügbar. Bei einer strengen Anonymisierungseinstellung ist dieser nicht verfügbar. Der Grund hierfür ist, dass der Bildverteilungsdienst ein Hochladen von Patientendaten von einem Krankenhaus in die Cloud erfordert und dies lediglich mit der Standard-Anonymisierungseinstellung möglich ist.

In Abhängigkeit der Anonymisierungseinstellung gibt es unterschiedliche Patientendatensätze, die von dem Krankenhaus in die Cloud durch die Hochladesoftware hochgeladen werden können. Zu diesem Zweck kann die Cloud auch einen Dienstparameter übermitteln, der anzeigt, welche Dienste in der Cloud bereitgestellt werden. Daneben kann auch ein Standort der Cloud übermittelt werden, beispielsweise in Form von geografischen Koordinaten oder einem Länderkürzel. Der Dienstparameter oder der Standort werden durch das Verfahren ausgewertet. Auf Basis dieser Auswertung wird automatisch eine Anonymisierungseinstellung gewählt, die Patientendatensätze erzeugt, die die erforderlichen Patientendaten umfassen.

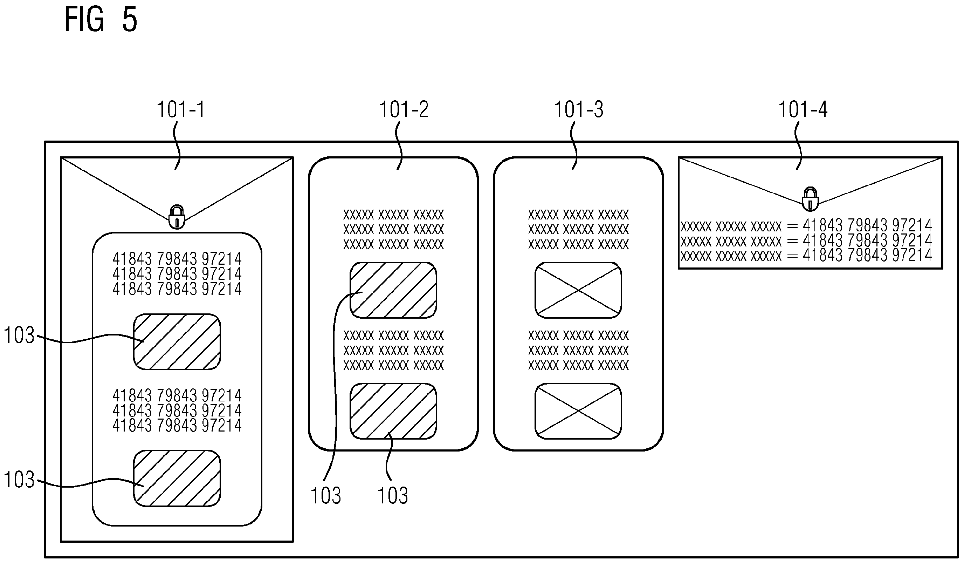

Fig. 5 zeigt eine schematische Ansicht unterschiedlicher Patientendatensätze 101-1, ... 101-4. Der Patientendatensatz 101-1 ist eine ursprüngliche DICOM-Datei, die verschlüsselt ist. Der Patientendatensatz 101-1 weist die Bilddateien 103 auf.

Der Patientendatensatz 101-2 ist eine DICOM-Datei mit ursprünglichen Bilddaten und Header-Daten, die mittels einer ersten vorgegebenen Anonymisierungseinstellung anonymisiert worden sind. Der Patientendatensatz 101-2 umfasst ebenfalls die Bilddaten 103.

Der Patientendatensatz 101-3 ist eine DICOM-Datei ohne Bilddaten und mit Header-Daten, die mittels einer zweiten vorgegebenen Anonymisierungseinstellung anonymisiert worden sind. Der Patientendatensatz 101-4 ist eine verschlüsselte Datei, die eine Abbildung zwischen den anonymisierten Headerdaten und den ursprünglichen Header-Daten umfasst.

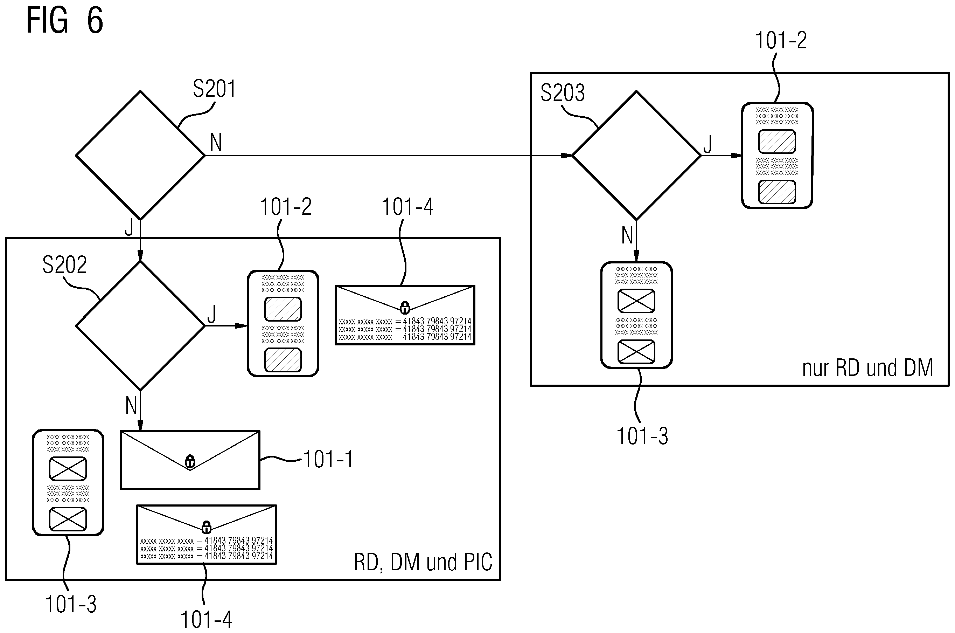

Fig. 6 zeigt eine Darstellung der Logik, die in einer Hochlade-Software implementiert ist und die auf das Hochladen der Patientendatensätze 101-1, ... 101-4 in Abhängigkeit der Anonymisierungseinstellung angewendet wird.

In Schritt S201 wird zunächst ermittelt, ob ein Bildverteilungsdienst von der Cloud bereitgestellt wird. Ist dies der Fall, wird anschließend in Schritt S102 ermittelt, ob ein Bilddatendienst bereitgestellt wird. Wird der Bilddatendienst bereitgestellt, werden die Patientendatensätze 101-2 und 101-4 übermittelt. Wird der Bilddatendienst nicht bereitgestellt, wird der Patientendatensatz 101-3 zusammen mit dem ursprünglichen Patientendatensatz 101-1 und dem Patientendatensatz 101-4 übermittelt. Eine Verarbeitung der Patientendatensätze 101-1, ... 101-4 kann durch den Bildverteilungsdienst, den Dosismanagement-Dienst oder den Scanner-Nutzungsdienst stattfinden. Falls der Bildverarbeitungsdienst aktiviert ist, weist dies auf die Standard-Anonymisierungseinstellung hin.

Wenn in Schritt S201 ermittelt wird, dass kein Bildverteilungsdienst von der Cloud bereitgestellt wird, wird anschließend in Schritt S103 ermittelt, ob ein Bilddatendienst bereitgestellt wird. Wird der Bilddatendienst bereitgestellt, wird der Patientendatensatz 101-2 übermittelt. Falls dies nicht der Fall ist, wird der Patientendatensatz 101-3 übermittelt. Eine Verarbeitung der Patientendatensätze 101-1 und 101-3 findet in diesem Fall durch den Dosismanagement-Dienst oder den Scanner-Nutzungsdienst statt. In folgenden sind Beispiele für Benutzerschnittstellen von Cloud-Diensten erläutert.

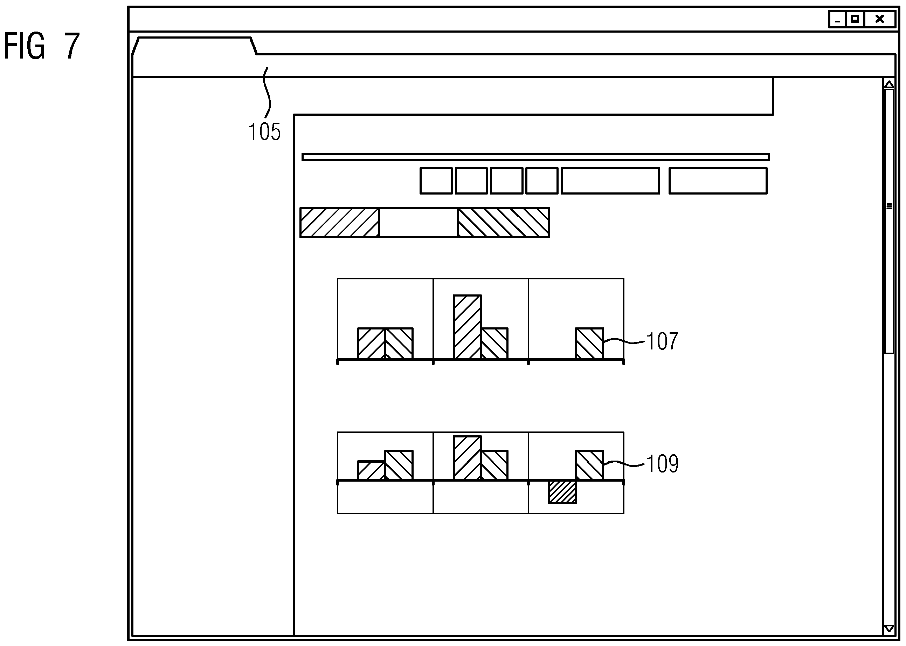

Fig. 7 zeigt ein Beispiel einer Benutzerschnittstelle 105 einer Scanner-Nutzung bei einer strengen Anonymisierungseinstellung. In dieser Benutzerschnittstelle 105 werden in Feld 107 die Dauer einer Untersuchung und in Feld 109 die Untersuchungen je Stunde für bestimmte Geräte angezeigt.

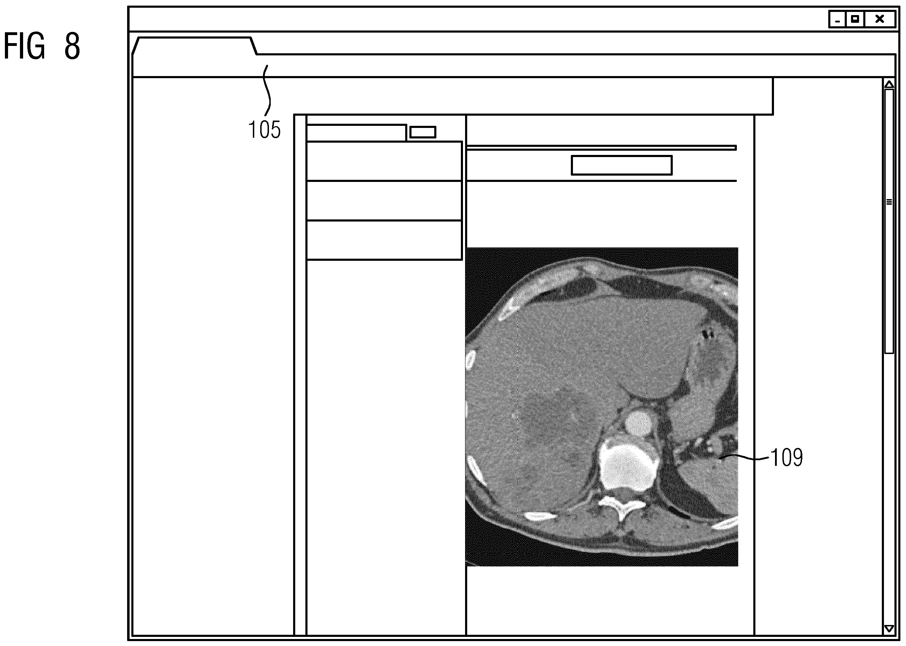

Fig. 8 zeigt ein Beispiel einer Benutzerschnittstelle 105 eines Bildverteilungsdienstes bei einer standardmäßigen Anonymisierungseinstellung. In Feld 109 werden Bilddaten angezeigt.

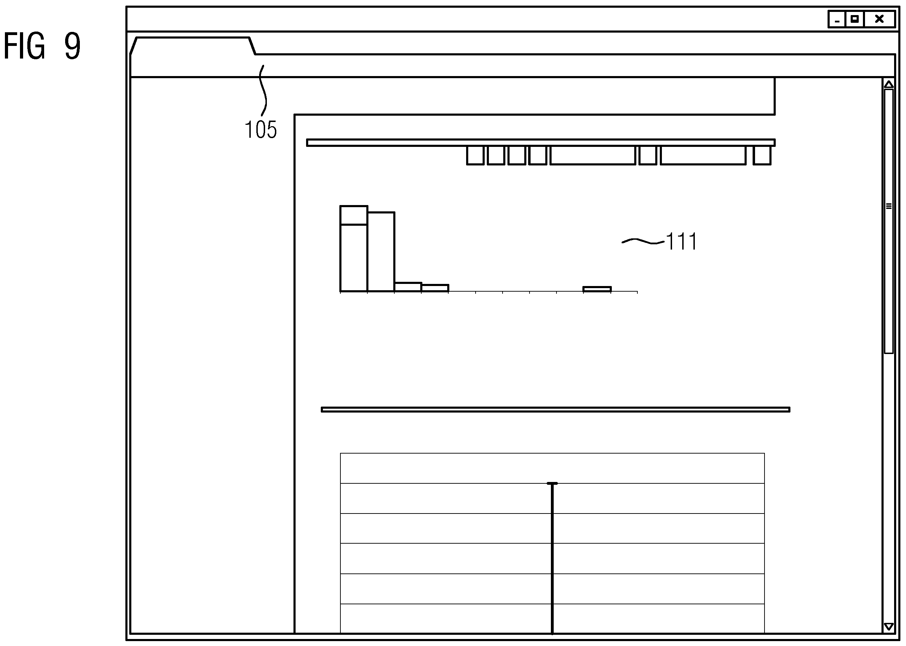

Fig. 9 zeigt ein Beispiel einer Benutzerschnittstelle 105 eines Dosismanagementdienstes bei einer sehr strengen Anonymisierungseinstellung. In Feld 111 wird der zeitliche Verlauf einer Dosis angezeigt.

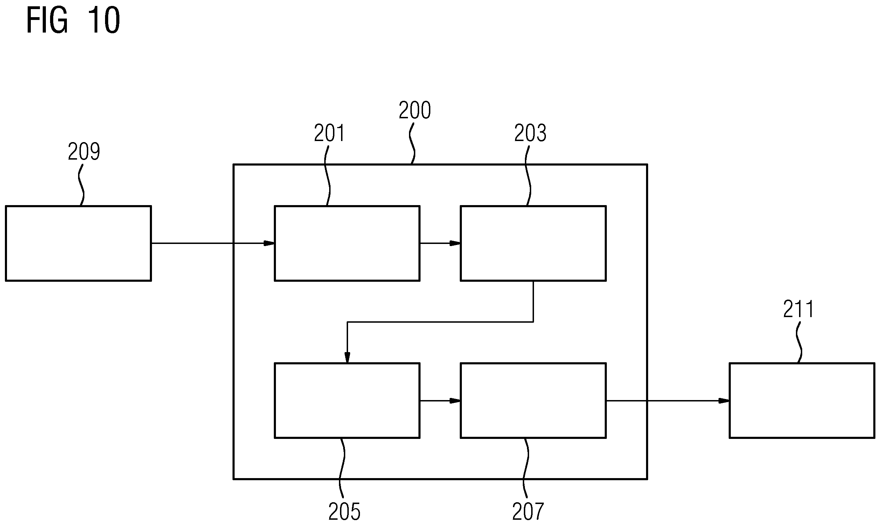

Fig. 10 zeigt ein Computersystem 200 zum Übertragen von medizinischen Datensätzen. Das Computersystem 200 umfasst eine Empfangseinrichtung 201 zum Empfangen eines Patientendatensatzes aus einem internen Datenspeicher 209. Eine Auswahleinrichtung 203 des Computersystems 200 dient zum Auswählen einer Anonymisierungseinstellung aus einem vorgegebenen Satz von Anonymisierungseinstellungen. Eine Erzeugungseinrichtung 205 dient zum Erzeugen des anonymisierten Patientendatensatzes 101-2, 101-3 auf Basis der ausgewählten Anonymisierungsregel. Die Sendeeinrichtung 207 dient zum Übertragen des anonymisierten Patientendatensatzes 101-2, 101-3 an einen externen Datenspeicher 211. Die Empfangseinrichtung 201, die Auswahleinrichtung 203, die Erzeugungseinrichtung 205 und die Sendeeinrichtung 207 können durch Software oder Hardware implementiert sein.

Das Verfahren verwendet einen Satz von Anonymisierungseinstellungen, die für unterschiedliche Länder weltweit angepasst sein können. Daneben wird ein Satz von Eigenschaften häufig verwendeter Cloud-Dienste verwendet, die für jede Anonymisierungseinstellung definiert sind. Dadurch ist die Möglichkeit gegeben, eine Anonymisierungseinstellung während einem Software-Einsatz in einer medizinischen Einrichtung in Abhängigkeit des Einsatzortes auswählen. Zudem ist die Möglichkeit gegeben, die Daten in allen Cloud-Diensten ungeachtet der verwendeten Anonymisierungseinstellung zu verarbeiten. Eine Konsistenz zwischen den Anonymisierungseinstellungen und der Fähigkeit der Cloud-Dienste, die hochgeladenen Daten zu verarbeiten, wird durch die technische Gestaltung sichergestellt. Weiter besteht die Möglichkeit die Anonymisierungseinstellungen zurückzusetzen. Ein Zurücksetzen der Anonymisierungseinstellungen kann sich als nützlich erweisen, falls eine medizinische Einrichtung die Anzahl von Diensten erhöht oder verringert, die in der Cloud verwendet werden.

Falls sich zum Beispiel ein Krankenhaus dazu entscheidet, einen Bildverteilungsdienst in der Cloud zu verwenden, wendet dieses neue Anonymisierungseinstellung im Vergleich zu jenen an, die zuvor verwendet worden sind, da der Bildverteilungsdienst ein Hochladen von Bilddaten in die Cloud voraussetzt. Die Auswahl und Übernahme neuer Anonymisierungsregeln kann durch einen Mausklick erfolgen, da weitere Anpassungen des Patientendatensatzes automatisch vorgenommen werden.

Das Heraufladen der Patientendatensätze wird automatisch durchgeführt und die Eigenschaften eines Dosismanagement-Dienstes (Dose Management Service), Dienstnutzungsdienstes (Service Utilization Service) und eines Bildverteilungsdienstes (Image Sharing Service) werden automatisch an die Einstellung angepasst.

Das Verfahren steht im Gegensatz zu existierenden Ansätzen, bei denen das Krankenhaus die Patientendatensätze nicht automatisch anhand einer Anonymisierungseinstellung verändern kann. In diesem Fall kann das Funktionieren von bereitgestellten Verarbeitungsdiensten nicht sichergestellt werden.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Ansprüche gegeben und wird durch die in der Beschreibung erläuterten oder den Figuren gezeigten Merkmale nicht beschränkt.

### Bezugszeichenliste

- 101: Patientendatensatz
- 103: Bilddaten
- 105: Benutzerschnittstelle
- 107: Feld
- 109: Feld
- 111: Feld
- 200: Computersystem
- 201: Empfangseinrichtung
- 203: Auswahleinrichtung
- 205: Erzeugungseinrichtung
- 207: Sendeeinrichtung
- 209: Datenspeicher
- 211: Datenspeicher

## Patentansprüche

1. Verfahren zum Übertragen von medizinischen Datensätzen, mit den Schritten:
Empfangen (S101) eines Patientendatensatzes (101-1) aus einem internen Datenspeicher (209);
Seitens eines externen Datenspeichers (211): Übermitteln eines Standortes des externen Datenspeichers (211);
Seitens des externen Datenspeichers (211): Übermitteln eines Dienstparameters des externen Datenspeichers (211), der anzeigt, welche Dienste von dem externen Datenspeicher (211) bereitgestellt werden;
automatisches Auswählen (S102) einer Anonymisierungseinstellung aus einem Satz von vorgegebenen Anonymisierungseinstellungen auf Basis des Standortes und des Dienstparameters des externen Datenspeichers (211);
Erzeugen (S103) eines anonymisierten Patientendatensatzes (101-2, 101-3) auf Basis der ausgewählten Anonymisierungseinstellung; und
Übertragen (S104) des anonymisierten Patientendatensatzes (101-2, 101-3) an den externen Datenspeicher (211).

2. Verfahren nach Anspruch 1, wobei der anonymisierte Patientendatensatz (101-2, 101-3) in dem externen Datenspeicher (211) gespeichert wird.

3. Verfahren nach einem der vorangehenden Ansprüche, wobei der empfangene und/oder der anonymisierte Patientendatensatz (101-1, 101-2, 101-3) eine DICOM-Datei ist.

4. Verfahren nach einem der vorangehenden Ansprüche, wobei die Anonymisierungseinstellung in den anonymisierten Patientendatensatz (101-2, 101-3) eingebettet wird.

5. Verfahren nach einem der vorangehenden Ansprüche, wobei der anonymisierte Patientendatensatz (101-2, 101-3) in dem internen Datenspeicher (209) gespeichert wird.

6. Verfahren nach einem der vorangehenden Ansprüche, wobei die Daten des Patientendatensatzes (101-1), die beim Erzeugen des anonymisierten Patientendatensatzes (101-2, 101-3) anonymisiert werden, in einem zusätzlichen nicht-anonymisierten Patientendatensatz (101-4) gespeichert werden.

7. Verfahren nach Anspruch 6, wobei eine Abbildung zwischen dem nicht-anonymisierten Patientendatensatz (101-4) und dem anonymisierten Patientendatensatz (101-2, 101-3) erzeugt wird.

8. Verfahren nach Anspruch 7, wobei die Abbildung verschlüsselt gespeichert wird.

9. Verfahren nach einem der vorangehenden Ansprüche, wobei der anonymisierte Patientendatensatz (101-2, 101-3) vor einem Übertragen verschlüsselt wird.

10. Computersystem (200) zum Übertragen von medizinischen Datensätzen, mit:
einer Empfangseinrichtung (201) zum Empfangen eines Patientendatensatzes (101-1) aus einem internen Datenspeicher (209);
einer Auswahleinrichtung (203) zum Auswählen einer Anonymisierungseinstellung aus einem vorgegebenen Satz von Anonymisierungseinstellungen auf Basis eines Standortes des externen Datenspeichers (211) und auf Basis eines Dienstparameters des externen Datenspeichers (211), der anzeigt, welche Dienste von dem externen Datenspeicher (211) bereitgestellt werden;
einer Erzeugungseinrichtung (205) zum Erzeugen eines anonymisierten Patientendatensatzes (101-2, 101-3) auf Basis der ausgewählten Anonymisierungsregel; und
einer Sendeeinrichtung (207) zum Übertragen des anonymisierten Patientendatensatzes (101-2, 101-3) an einen externen Datenspeicher (211).

## Claims

1. Method for transmitting medical data records, having the steps:
receiving (S101) a patient data record (101-1) from an internal data storage unit (209);
on the part of an external data storage unit (211): transferring a location of the external data storage unit (211);
on the part of the external data storage unit (211):
transferring a service parameter of the external data storage unit (211) which indicates which services are provided by the external data storage unit (211);
automatically selecting (S102) an anonymisation setting from a set of predetermined anonymisation settings based on the location and the service parameter of the external data storage unit (211);
generating (S103) an anonymised patient data record (101-2, 101-3) based on the selected anonymisation setting; and transmitting (S104) the anonymised patient data record (101-2, 101-3) to the external data storage unit (211).

2. Method according to claim 1, wherein the anonymised patient data record (101-2, 101-3) is stored in the external data storage unit (211).

3. Method according to one of the preceding claims, wherein the received and/or the anonymised patient data record (101-1, 101-2, 101-3) is a DICOM file.

4. Method according to one of the preceding claims, wherein the anonymisation setting is embedded in the anonymised patient data record (101-2, 101-3).

5. Method according to one of the preceding claims, wherein the anonymised patient data record (101-2, 101-3) is stored in the internal data storage unit (209).

6. Method according to one of the preceding claims, wherein the data of the patient data record (101-1), which is anonymised when the anonymised patient data record (101-2, 101-3) is generated, is stored in an additional non-anonymised patient data record (101-4).

7. Method according to claim 6, wherein a mapping between the non-anonymised patient data record (101-4) and the anonymised patient data record (101-2, 101-3) is generated.

8. Method according to claim 7, wherein the mapping is stored encrypted.

9. Method according to one of the preceding claims, wherein the anonymised patient data record (101-2, 101-3) is encrypted prior to transmission.

10. Computer system (200) for transmitting medical data records, having:
a receiving facility (201) for receiving a patient data record (101-1) from an internal data storage unit (209);
a selection facility (203) for selecting an anonymisation setting from a predetermined set of anonymisation settings based on a location of the external data storage unit (211) and based on a service parameter of the external data storage unit (211), which indicates which services are provided by the external data storage unit (211);
a generation facility (205) for generating an anonymised patient data record (101-2, 101-3) on the basis of the selected anonymisation rule; and
a transmit facility (207) for transmitting the anonymised patient data record (101-2, 101-3) to an external data storage unit (211).

## Revendications

1. Procédé de transmission de jeux de données médicales, comprenant les stades :
réception (S101) d'un jeu (101-1) de données de patient, à partir d'une mémoire (209) interne de données;
de la part d'un mémoire (211) externe de données : transmission d'un site de la mémoire (211) externe de données;
de la part de la mémoire (211) externe de données : transmission d'un paramètre de service de la mémoire (211) externe de données, qui indique les services que la mémoire (211) externe de données procure;
sélection (S102) automatique d'un réglage d'anonymisation, à partir d'un jeu de réglages d'anonymisation donné à l'avance, sur la base du site et du paramètre de service de la mémoire (211) externe de données;
production (S103) d'un jeu (101-2, 101-3) de données de patient anonymisé sur la base du réglage d'anonymisation sélectionné et
transmission (S104) du jeu (101-2, 101-3) de données de patient anonymisé à la mémoire (211) externe de données.

2. Procédé suivant la revendication 1, dans lequel on met le jeu (101-2, 101-3) de données de patient anonymisé en mémoire dans la mémoire (211) externe de données.

3. Procédé suivant l'une des revendications précédentes, dans lequel le jeu (101-1, 101-2, 101-3) de données de patient reçu et/ou anonymisé est un fichier DICOM.

4. Procédé suivant l'une des revendications précédentes, dans lequel on incorpore le réglage d'anonymisation dans le jeu (101-2, 101-3) de données de patient anonymisé.

5. Procédé suivant l'une des revendications précédentes, dans lequel on met le jeu (101-2, 101-3) de données de patient anonymisé dans la mémoire (209) interne de données.

6. Procédé suivant l'une des revendications précédentes, dans lequel on met en mémoire, dans un jeu (101-4) de données de patient supplémentaire non anonymisé, les données du jeu (101-1) de patient, qui ont été anonymisées lors de la production du jeu (101-2, 101-3) de données de patient anonymisé.

7. Procédé suivant la revendication 6, dans lequel on produit une relation entre le jeu (101-4) de données de patient non anonymisé et le jeu (101-2, 101-3) de données de patient anonymisé.

8. Procédé suivant la revendication 7, dans lequel on mémorise la relation, de manière chiffrée.

9. Procédé suivant l'une des revendications précédentes, dans lequel on chiffre le jeu (101-2, 101-3) de données de patient anonymisé avant une transmission.

10. Système (200) d'ordinateur pour transmettre des jeux de données médicales, comprenant :
un dispositif (201) de réception pour recevoir un jeu (101-1) de données de patient à partir d'une mémoire (209) interne de données; un dispositif (203) de sélection pour sélectionner un réglage d'anonymisation à partir d'un jeu donné à l'avance de réglageS d'anonymisation sur la base d'un site de la mémoire (211) externe de données et sur la base d'un paramètre de service de la mémoire (211) externe de données, qui indique les services procurés par la mémoire (211) externe de données;
un dispositif (205) de production pour produire un jeu (101-2, 101-3) de données de patient anonymisé sur la base de la règle d'anonymisation sélectionnée et
un dispositif (207) d'émission pour transmettre le jeu (101-2, 101,3) de données de patient anonymisé à la mémoire (211) externe de données.
